# EUROPÄISCHE PATENTSCHRIFT

(11) **EP 0 998 949 B1**
(45) Veröffentlichungstag und Bekanntmachung des Hinweises auf die Patenterteilung: **04.02.2004**
(21) Anmeldenummer: 99121382.8
(22) Anmeldetag: 27.10.1999
(51) Int. Cl.: A61L 26/00, A61L 15/32, A61P 17/02

(54) **Flexible Wundauflage auf Fibrinbasis und Verfahren zu ihrer Herstellung**
Flexible wound dressing based on fibrin and process for preparation of same
Pansement fexible à base de fibrine et procédé de préparation

(30) Priorität: 06.11.1998 DE 19851334
(43) Veröffentlichungstag der Anmeldung: 10.05.2000
(73) Patentinhaber: Aventis Behring Gesellschaft mit beschränkter Haftung, 35002 Marburg (DE)
(72) Erfinder: Rapp, Mirna, Dr., 35037 Marburg (DE)

(56) Entgegenhaltungen:
- EP-A- 0 068 149
- EP-A- 0 090 997
- DE-A- 3 037 513
- US-A- 5 407 671

## Beschreibung

Gegenstand der Erfindung ist ein flexibles, biologisch abbaubares Fibrinvlies für die Wundheilung und seine Herstellung. Derartige Vliesmaterialien können zur Hämostase auf inneren und äusseren Wunden sowie als Trägermaterialien für biologische Faktoren eingesetzt werden.

Es ist bekannt, daß chirurgische Eingriffe auf innere Organe von Problemen des Wundverschlusses begleitet sind, bedingt durch die starke Durchblutung und Weichheit des Gewebes. Eine häufig angewendete Methode zum Verschluss innerer Wunden ist die Elektrokoagulation der Blutgefässe, die in den meisten Fällen zu für den Patienten schmerzhaften postoperativen Adhäsionen führt. Ausserdem ist sie zum Verschliessen von posttraumatischen Organrupturen nur begrenzt geeignet. Eine gute Alternative stellt der Fibrinkleber dar, mit dem die Wunde durch vorangehende Reaktion von mehreren körpereigenen, humanen Gerinnungsfaktoren verschlossen wird. Das auf der Wunde dabei entstehende Gerinnsel zeichnet sich durch eine hohe Verträglichkeit und rasche Blutstillung aus. Weniger vorteilhaft ist die der Anwendung des Fibrinklebers vorangehende längere Vorbereitungszeit sowie die etwas umständliche Anwendung, vor allem in der minimal-invasiven Chirurgie, wo ein spezieller Applikator zum Endoskop benötigt wird. Es hat deshalb auch schon Versuche gegeben, eine biologisch abbaubare Wundauflage auf Fibrinbasis zu entwickeln, die gebrauchsfertig zur Verfügung gestellt wird und ohne besondere Vorbereitungen auf die Wunde appliziert werden kann. Ein derartiges Vliesmaterial ist aus der europäischen Patentanmeldung 0 068 149 bekannt, die auf ein fibrinogenhaltiges Trockenpräparat gerichtet ist, das eine durch Gefriertrocknung erzielte Schaum- oder Vliesstruktur aufweist und neben Thrombin in zumindest katalytisch wirksamen Mengen im wesentlichen aus etwa 10 bis 95 Gewichtsprozent Fibrin und etwa 5 bis 90 Gewichtsprozent Fibrinogen besteht. Bei der Herstellung einer vliesartigen Wundauflage wird dabei zunächst in einer Fibrinogen und Thrombin enthaltenden wässrigen Lösung Fibrin erzeugt und das entstehende Reaktionsgemisch tiefgefroren und lyophilisiert. Dieses Material ist vor allem als Wundversorgungsmaterial, als Füllmaterial für Knochenhohlräume und/oder als Trägermaterial für weitere Wirkstoffe vorgesehen. Ein gravierender Nachteil dieser Wundauflage besteht allerdings darin, dass es sich hierbei um ein starres und brüchiges Vlies handelt, das auch nach Kontakt mit Wasser oder Blut seine Starrheit bewahrt. Dadurch sind die Anwendungsmöglichkeiten eines derartigen Fibrinvlieses äusserst beschränkt. Außerdem ist aus der DE-PS 32 14 337 bereits ein resorbierbarers, mehrschichtiges Flächenmaterial bekannt, dass wenigstens eine thrombinfreie und wenigens eine fibrinogenfreie Teilschicht enthält, die jeweils eine Glykoprotein-Matrix aufweisen. Durch die räumliche Trennung von Thrombin und Fibrinogen wird dabei die Bildung eines Fibrinvlieses verhindert.

Es hat deshalb auch nicht an Versuchen gefehlt, aus anderen biologischen Materialien geeignetere Wundauflagen zu entwickeln. Im Handeln befinden sich Vliese, die aus einem biologischen Material wie Kollagen bestehen, welches hauptsächlich aus tierischem Bindegewebe vom Rind, vom Pferd oder vom Schwein gewonnen wird. So ist beispielsweise in der EP-A 0 485 210 eine fibrinhaltige Kollagenmembran vorgeschlagen worden.

Derartige Materialien bedürfen jedoch einer Virusinaktivierung und besonderer Vorsichtsmassnahmen, um eine potentielle Übertragung der BSE-Erreger zu vermeiden. Ausserdem wurde in der Literatur über Fälle von Patienten mit Überempflindlichkeitsreaktionen gegen Rinderkollagen berichtet. Deshalb ist auch schon daran gedacht worden, Kollagenvliese aus humanem Material, zum Beispiel aus Kollagen, das aus der Plazenta gewonnen wird, zur Verfügung zu stellen. Das könnte zwar eine bessere Bioverträglichkeit beinhalten, ist jedoch ebenfalls mit dem Problem der Virussicherheit belastet. Es ist deshalb auch schon der Gedanke vorgetragen worden, rekombinantes oder transgenes Human-Kollagen zur Herstellung von Wundauflagen einzusetzen, jedoch ist derartiges Kollagen beim heutigen Stand der Entwicklung schon aus Kostengründen noch nicht konkurrenzfähig. Schliesslich ist auch aus der US-PS 2 492 458 ein Fibrinschaum bekannt, der zur Blutstillung permanent in einer Wunde eingelegt werden kann.

Schliesslich ist auch schon daran gedacht worden, synthetisch hergestellte, biologisch abbaubare Materialien wie Polyhydroxycarbonsäuren oder Polyaminosäuren zur Herstellung von abbaubaren Wundauflagen einzusetzen. Diese Materialien bieten zwar eine preiswertere Alternative zum rekombinanten Kollagen, jedoch ist ihre Anwendung beschränkt, da sie sich für die Hämostase bei stark blutenden und weichen Geweben wenig eignen. Zudem sind sie schlechter verträglich, da bei ihrer Anwendung durch Abbauvorgänge Produkte entstehen, die Entzündungsreaktionen im Körper hervorrufen können.

Es stelle sich deshalb die Aufgabe, eine biologisch abbaubare Wundauflage zu entwickeln, die die bekannten Nachteile der bisher üblichen Wundauflagen vermeidet. Gelöst wird diese Aufgabe erfindungsgemäss durch eine biologisch abbaubare Wundauflage auf Fibrinbasis, die durch besondere, bei ihrer Herstellung angewendete Verfahrensschritte eine hohe Flexibilität aufweist und sich deshalb hervorragend als Vlies zur Abdeckung und zum Verschluss von blutenden Wunden eignet.

Es wurde nun gefunden, dass diesen Anforderungen eine biologisch abbaubare, flexible Wundauflage auf Fibrinbasis entspricht, bei der die Wundauflage ein Fibrinvlies ist oder ein Fibrinvlies enthält, das von dialysierbaren Bestandteilen weitgehend oder vollständig befreit ist. Dabei ist das Fibrinvlies vorzugsweise mit einem biologisch abbaubaren Trägermaterial fest verbunden. Besonders geeignet ist ein Fibrinvlies, das 10-80 Gewichtsprozent Glyzerin pro Fibrinmenge enthält.

Eine derartige Wundauflage auf Fibrinbasis kann erfindungsgemäß durch ein Verfahren hergestellt werden kann, bei dem eine Fibrinogenlösung einer einstufigen oder mehrstufigen Dialyse unterworfen wird, dann durch Einwirkung einer wässrigen Thrombinlösung auf die Fibrinogenlösung ein flexibles Fibrinvlies gebildet und dieses anschliessend einer Gefriertrocknung unterworfen wird.

Durch die einstufige oder mehrstufige Dialyse der Fibrinogenlösung wird deren Zusammensetzung entscheidend geändert und die in ihr üblicherweise enthaltenen Konzentrationen an Salzen und Aminosäuren erheblich vermindert: Es wird angenommen, dass das die entscheidende Voraussetzung zur Herstellung eines flexiblen Fibrinvlieses ist. Die Dialyse der Fibrinogenlösung kann auf verschiedene Weise durchgeführt werden. Bei einer einstufigen Lösung wird die Fibrinogenlösung in einem Dialysebad behandelt, das gleichzeitig einen organischen Komplexbildner und physiologisch verträgliche anorganische Salze enthält. Die einstufige Dialyse kann allerdings auch mit einem Dialysebad durchgeführt werden, das ausschliesslich organische Komplexbildner enthält. Dann wird nach Beendigung der Dialyse das physiologisch verträgliche anorganische Salz, in der Regel NaCl, der Fibrinogenlösung in einer Menge von 0,05 bis 0,5 Gewichtsprozent zugesetzt.

Bei der Dialyse werden als organische Komplexbildner vor allem Alkalisalze der Ethylendiamintetraessigsäure, der Oxalsäure oder der Zitronensäure zugesetzt. Die Konzentration des organischen Komplexbildners im Dialysebad beträgt im allgemeinen 1 bis 20 mM, vorzugsweise 1 bis 5 mM.

Es ist jedoch auch möglich, die Dialyse zweistufig durchzuführen. Dabei wird im ersten Dialyseschritt die Fibrinogenlösung mit einer wässrigen Lösung eines der vorstehend genannten Komplexsalze dialysiert und anschliessend in einem zweiten Dialysebad die Dialyse mit einer NaCl-Lösung oder der Lösung eines anderen physiologisch verträglichen anorganischen Salzes durchgeführt.

Das somit von dialysierbaren Begleitstoffen befreite Fibrinogen, das auch noch den in Fibrinkleber-Präparaten üblichen Zusatz von Faktor XIII enthalten kann, ist ein hervorragend geeigneter Ausgangsstoff für die Herstellung eines flexiblen Fibrinvlieses. Dieses wird durch Vermischen der dialysierten Fibrinogenlösung mit einer wässrigen Lösung aus Thrombin und Kalziumchlorid hergestellt. Dabei ist das wasserlösliche Kalziumsalz in der Regel in einer Menge von 0,5 bis 40 mM anwesend. Im allgemeinen wird zur Herstellung des Fibrinvlieses das Fibrinogen in einer Menge von 10 bis 25 g/l und das Thrombin in einer Menge von bis zu 100 I.E./ml eingesetzt. Die Herstellung des Fibrinvlieses geschieht bei einer Temperatur zwischen 0 und 37°C.

Zur Herstellung des Fibrinvlieses kann die Thrombin/Kalziumchloridlösung der Fibrinogenlösung zugemischt werden, jedoch ist es viel vorteilhafter, auf die in einer flachen Schale vorgelegte Fibrinogenlösung die Thrombin/Kalzium chloridlösung aufzusprühen. Zur Herstellung eines mehrere Meter breiten Fibrinvlieses wird auf die in einer entsprechend breiten Flüssigkeitswanne enthaltene Fibrinogenlösung die Thrombin/Kalziumchloridlösung aus in Reihen angeordneten Sprühköpfen aufgesprüht, die sich gleichmässig über die Fibrinogenlösung hinwegbewegen. Nach einer zur Ausbildung des Fibrinvlieses ausreichenden Reaktionszeit wird es anschliessend einer Trocknung, vorzugsweise Gefriertrocknung unterworfen.

Die Gefriertrocknung wird vorzugsweise in folgender Weise durchgeführt:

Das Fibrinvlies wird zwei Stunden bei -28°C eingefroren und anschließend zwölf Stunden im Vakuum bei derselben Temperatur getrocknet. Dann folgen zwei Trocknungsschritte bei -10°C und +20°C für jeweils 16 bzw. 8 Stunden ebenfalls im Vakuum.

Eine besonders vorteilhafte erfindungsgemäße Wundauflage auf Fibrinbasis lässt sich herstellen, wenn man das Fibrinvlies mit einem biologisch abbaubaren Trägermaterial kombiniert. Als hierfür geeignetes Trägermaterial kommen vor allem natürliches oder chemische modifiziertes Collagen, Keratin, Gelatine, Kohlenhydrate oder Zellulosederivate in Frage. Das Trägermaterial kann aber auch aus einem synthetischen, biologisch abbaubaren Polymeren bestehen. Geeignet sind u.a. Polyhydroxycarbonsäuren, Polyester, Polycyanoacrylate, Polyaminosäuren, Polyalkohole sowie Silikone. Diese Trägermaterialien können vorzugsweise als Vliesstoff oder als Gewebe eingesetzt werden. Besonders geeignet sind z.B. das im Handel befindliche Vlies Ethisorb® Patch Typ 6 der Firma Ethicon und das Collagenvlies Surgicoll der Firma Medical Biomaterial Products. Beim Ethisorb® Patch Typ 6 der Firma Ethicon handelt es sich um ein synthetisches, resorbierbares Vlies aus Polyglactin 910 (Vicryl®) und Poly-p-dioxanon (PDS). Polyglactin 910 ist ein Copolymer aus Glycolid und Lactid. Diese Vliese oder andere abbaubare Trägermaterialien lassen sich mit dem Fibrinvlies am besten während dessen Herstellung beschichten. Dabei geht man so vor, dass die Bildung des Fibrinvlieses in Gegenwart des Trägermaterials erfolgt. Dazu wird das Trägermaterial in die in einer flachen Schale enthaltene Fibrinogenlösung eingelegt, bis sie völlig mit der Flüssigkeit überdeckt ist. Dann wird eine Thrombin/Kalziumchloridlösung aufgesprüht, wodurch sich ein Fibrinvlies bildet, das sich auf dem Trägermaterial ablagert und auf ihm fest haftet. Dabei entsteht ein zweischichtiges Vlies mit hoher Flexibilität, das sich ohne Deformation der Beschichtungen biegen lässt. Die Herstellung einer derartigen mehrschichtigen Wundauflage auf Fibrinbasis hat den Vorteil einer Verbesserung des hämostatischen Effektes bei stark blutenden Wunden. Die Flexibilität des Trägermaterials wird dabei durch die Fibrinvliesauflage nicht beeinträchtigt, da das Fibrinvlies sehr flexibel ist, wenn es nach dem erfindungsgemäßen Verfahren hergestellt worden ist.

Das erfindungsgemässe Fibrinvlies kann in seinem therapeutischen Wert noch verbessert werden, wenn ihm die Wundheilung fördernde biologische, pflanzliche oder synthetische Wirkstoffe wie Immunglobuline, Chemotherapeutika oder Antibiotika zugesetzt werden. Es ist zweckmässig, diese Substanzen schon der Fibrinogenlösung zuzusetzen, damit sie in dem durch den Thrombinzusatz entstehenden Fibrinvlies integriert sind. Diese Substanzen können allerdings auch auf das fertige Fibrinvlies durch Aufsprühen aufgetragen werden.

Eine bemerkenswerte Verbesserung des erfindungsgemässen Fibrinvlieses bezüglich ihrer Reissfestigkeit und Elastizität lässt sich durch Zugabe von Glyzerin zu der Fibrinogenlösung erreichen. Gibt man der Fibrinogenlösung vor der Fibrinbildung Glyzerin in einer prozentualen Menge zwischen 0,2 und 1,0 Volumenprozent (v/v) zu, dann erhält man eine Fibrinwundauflage, die 10 bis 80 Gew.% Glyzerin pro Fibrinmenge, vorzugsweise 20 bis 50 Gewichtsprozent Glyzerin pro Fibrinmenge enthält. Die Reisskraft und Elastizität des so durch den Glyzerinzusatz modifizierten Fibrinvlieses ist so erheblich, dass das Fibrinvlies eine gummiartige Konsistenz erhält. Der Glyzerinzusatz hat darüber hinaus auch den Vorteil, dass er keine chemische Reaktion mit den Gerinnungsfaktoren eingeht. Im Gegensatz dazu kann bei Wundauflagen auf Kollagenbasis eine Erhöhung der biomechanischen Stabilität durch chemische Quervernetzungen erreicht werden, was jedoch eine Ausbildung von potentiellen Neoantigenen und somit schlechterer Bioverträglichkeit zur Folge haben kann.

Die erfindungsgemässen Wundauflagen auf Fibrinbasis lassen sich zur gewünschten Grösse zuschneiden, zusammenrollen und wieder aufrollen. Auf diese Weise sind sie auch in der minimal-invasiven Chirurgie sehr gut einsetzbar.

Die Erfindung wird durch die nachfolgenden Beispiele näher erläutert:

### Beispiel 1:

Fibrinogen (Beriplast®) wurde in Wasser aufgenommen und in 0,02 M Natriumzitrat dialysiert. Anschliessend erfolgte eine Dialyse in 0,05% NaCl. Das Dialysat wurde mit 15% Glyzerin versetzt und in vorgegebene Metallwannen gegossen. Anschliessend wurde auf das Gemisch eine Thrombinlösung aufgesprüht und dann die Mischung bei Raumtemperatur 30 bis 60 Minuten inkubiert. Das entstandene Fibringerinnsel wird bei -28°C eingefroren und in folgender Weise gefriergetrocknet Zunächst wird das Fibrinvlies 12 Stunden bei -28°C eingefroren und anschließend 12 Stunden im Vakuum bei derselben Temperatur getrocknet. Dann folgen zwei Trocknungsschritte bei -10°C für 16 Stunden und bei +25°C für 8 Stunden, wobei ein Vakuum von 1,35 x 10⁻¹ mbar angelegt wird.

### Beispiel 2:

Fibrinogen (Beriplast®) wurde in Wasser aufgenommen und in 2 mM Natriumzitrat dialysiert. Anschliessend erfolgte eine Dialyse in 0,05% Natriumchlorid. Das Dialysat wurde dann wie im Beispiel 1 weiterverarbeitet und nach Inkubation mit Thrombinlösung und anschliessender Gefriertrocknung ein flexibles Fibrinvlies erhalten.

### Beispiel 3:

Fibrinogen (Beriplast®) wurde in Wasser aufgenommen und in 2 mM Natriumzitrat dialysiert. Anschliessend wurde eine Dialyse in 0,1% Natriumchlorid durchgeführt. Danach wurde durch Aufsprühen einer Thrombinlösung, Inkubation bis zu 60 Minuten und anschliessender Gefriertrocknung ein flexibles Fibrinvlies erhalten.

### Beispiel 4:

Fibrinogen (Beriplast®) wurde in Wasser aufgenommen und in einer 2mM Natriumzitratlösung dialysiert. Danach wurde so viel von einer 10%-igen Natriumchloridlösung zugesetzt, bis die Fibrinogenlösung 0,05% Natriumchlorid enthielt. Dann wurde die weitere Herstellung des Fibrinvlieses entsprechend den vorstehenden Beispielen durchgeführt.

### Beispiel 5:

Fibrinogen (Beriplast®) wurde in Wasser aufgenommen und in 2 mM Natriumzitrat und 0,05 bis 0,07% Natriumchlorid dialysiert. Aus dieser Fibrinogenlösung wurde durch Thrombinzusatz und anschliessende Gefriertrocknung das erfindungsgemässe Fibrinvlies wie in den vorstehenden Beispielen beschrieben hergestellt.

### Beispiel 6:

Zur Herstellung des Fibrinvlieses wurden der Fibrinogenlösung vor der Fibrinbildung unterschiedliche Mengen an Glyzerin in Prozent (v/v) zugesetzt und anschliessend die Reisskraft und die Elastizität der Vliese im trockenen und nassen Zustand gemessen. Die Ergebnisse sind in der folgenden Tabelle wiedergegeben:

**Tabelle:**

| **Zusatz an Glyzerin in % (v/v)** | **Reisskraft (N)** | | **Elastizität (mm)** | |
|---|---|---|---|---|
| | **Vliese trocken** | **Vliese nass** | **Vliese trocken** | **Vliese nass** |
| 0 | 1.5 | 0,6 | 3.6 | 28.1 |
| 0.2 | 2.0 | 0.5 | 11.8 | 33.9 |
| 0.4 | 1.8 | 1.1 | 16.1 | 36.4 |
| 0.8 | 2.3 | 1.5 | 25.7 | 48.7 |

Die Werte der Tabelle zeigen, dass der Zusatz von Glyzerin konzentrationsabhängig die mechanische Stabilität und Dehnbarkeit der Fibrinvliese erhöht Diese Fibrinvliese eignen sich zur Anwendung in der Chirurgie an inneren Organen und bieten Einsatzmöglichkeiten in der Endoskopie.

### Beispiel 7:

Fibrinogen (Beriplast®) wurde in Wasser aufgenommen und in 2 mM Natriumzitrat dialysiert. Anschliessend erfolgte eine Dialyse in 0,07% NaCl. Das Dialysat wurde mit 15% Glyzerin versetzt. In eine 5 x 5 cm Metallwanne wird das Vlies Ethisorb® Patch Typ 6 (Ethicon GmbH) auf dem Wannenboden angeheftet, worauf das Dialysat gegossen und die Fibrinbildung durch das Aufsprühen von Thrombin induziert wurde. Nach der Gefriertrocknung resultierte ein biegsames, zweischichtiges Vlies, bei dem beide Schichten gut aneinander hafteten.

## Patentansprüche

1. Flexible Wundauflage auf Fibrinbasis, **dadurch gekennzeichnet, dass** die Wundaufkage ein Fibrinvlies enthält, das aus einem Fibrinogen herstellbar ist, das herstellbar ist durch ein Verfahren, in dem das Fibrinogen in einer... einstufigen oder mehrstufigen Dialyse gegen ein physiologisch verträgliches anorganisches Salz im Dialysebad in einer Menge von bis zu 0,5 Gewichtsprozent von dialysierbaren Bestandteilen weitgehend oder vollständig befreit.

2. Flexible Wundauflage nach Anspruch 1, **dadurch gekennzeichnet, dass** das Fibrinvlies mit einem biologisch abbaubaren Trägermaterial fest verbunden ist.

3. Flexible Wundauflage nach den Ansprüchen 1 und 2, **dadurch gekennzeichnet, dass** das Fibrinvlies 10 bis 80 Gew.-% Glyzerin pro Fibrinmenge enthält.

4. Verfahren zur Herstellung einer flexiblen Wundauflage auf Fibrinbasis, **dadurch gekennzeichnet, dass** man eine Fibrinogenlösung einer einstufigen oder mehrstufigen Dialyse gegen ein physiologisch verträgliches anorganisches Salz im Dialysebad in einer Menge von bis zu 0,5 Gewichtsprozent unterwirft, dann durch Einwirkung einer wässrigen Thrombinlösung, die ein wasserlösliches Kalziumsalz enthält, auf die Fibrinogenlösung ein flexibles Fibrinvlies bildet und dieses anschliessend einer Gefriertrocknung unterwirft.

5. Verfahren nach Anspruch 4, **dadurch gekennzeichnet, dass** die Bildung des Fibrinvlieses in Gegenwart eines biologisch abbaubaren Trägermaterials erfolgt, auf dem sich das Fibrinvlies als festhaftende Schicht niederschlägt.

6. Verfahren nach Anspruch 5, **dadurch gekennzeichnet, dass** das bioabbaubare Trägermaterial aus natürlichen oder chemisch modifiziertem Collagen, Keratin, Gelatine, Kohlenhydraten oder Zellulosederivaten besteht.

7. Verfahren nach Anspruch 5, **dadurch gekennzeichnet, dass** das bioabbaubare Trägermaterial aus einem Polymer aus der Gruppe der Polyhydroxycarbonsäuren, der Polyester, der Polycyanoacrylate, der Polyaminosäuren, der Polyalkohole oder der Silikone besteht.

8. Verfahren nach Ansprüchen 4 bis 7, **dadurch gekennzeichnet, dass** die Fibrinogenlösung auch noch den Faktor XIII enthält.

9. Verfahren nach den Ansprüchen 4 bis 8, **dadurch gekennzeichnet, dass** man die Fibrinogenlösung einstufig mit einer einen Komplexbildner und ein physiologisch verträgliches anorganisches Salze enthaltenden Lösung dialysiert.

10. Verfahren nach den Ansprüchen 4 bis 8, **dadurch gekennzeichnet, dass** man die Fibrinogenlösung einstufig mit einer einen Komplexbildner enthaltenden Lösung dialysiert und anschliessend der Fibrinogenlösung ein physiologisch verträgliches anorganisches Salz zusetzt.

11. Verfahren nach den Ansprüchen 4 bis 8, **dadurch gekennzeichnet, dass** man die Dialyse zweistufig durchführt, wobei die Fibrinogenlösung zunächst mit einer Lösung eines Komplexbildners und anschliessend mit einer Lösung eines physiologisch verträglichen anorganischen Salzes dialysiert wird.

12. Verfahren nach den Ansprüchen 4 bis 11, **dadurch gekennzeichnet, dass** als Komplexbildner eine wässrige Lösung eines Alkalisalzes der Ethylendiaminessigsäure (EDTA), der Oxalsäure oder der Zitronensäure zur Dialyse der Fibrinogenlösung eingesetzt wird.

13. Verfahren nach den Ansprüchen 9 bis 12, **dadurch gekennzeichnet, dass** die Konzentration des Komplexbildners im Dialysebad 1 bis 20 mM, vorzugsweise 1 bis 5 mM beträgt.

14. Verfahren nach den Ansprüchen 9 bis 11, **dadurch gekennzeichnet, dass** als physiologisch verträgliches anorganisches Salz NaCl eingesetzt wird.

15. Verfahren nach Anspruch 4, **dadurch gekennzeichnet, dass** das wasserlösliche Kalziumsalz in einer Menge von 0,5 bis 40 mM zugesetzt wird.

16. Verfahren nach den Ansprüchen 4 bis 15, **dadurch gekennzeichnet, dass** das Fibrinogen in einer Menge von 10 bis 25 g/l und das Thrombin in einer Menge von weniger als 1000 I.E./ml eingesetzt wird.

17. Verfahren nach den Ansprüchen 4 bis 16, **dadurch gekennzeichnet, dass** dem Fibrinvlies die Wundheilung fördernde Wirkstoffe zugesetzt werden.

18. Verfahren nach den Ansprüchen 4 bis 16, **dadurch gekennzeichnet, dass** bei der Herstellung des Fibrinvlieses dem Fibrinogen Glyzerin in einer Menge von 0,2 bis 1,0 Volumenprozent zugesetzt wird.

19. Flexible Wundauflage auf Fibrinbasis, **dadurch gekennzeichnet, dass** sie nach den Ansprüchen 4 bis 18 erhältlich ist.

20. Verwendung einer Wundauflage nach den Ansprüchen 1 bis 3 und 19 zur Herstellung eines Arzneimittels zur Hämostase innerer und äußerer Wunden.

21. Verwendung einer Wundauflage nach den Ansprüchen 1 bis 3 und 19 zur Herstellung eines Trägermaterials für die Wundheilung fördernde Wirkstoffe.

22. Trägermaterial für die Wundheilung fördernde Wirkstoffe, herstellbar aus einer Wundauflage nach den Ansprüchen 1 bis 3 und 19.

## Claims

1. A flexible wound covering based on fibrin, **characterized in that** the wound covering contains a fibrin web which is preparable from a fibrinogen which is preparable by a process in which the fibrinogen is largely or completely freed of dialyzable constituents in a single-stage or multistage dialysis against a physiologically compatible inorganic salt in the dialysis bath in an amount of up to 0.5 percent by weight.

2. A flexible wound covering as claimed in claim 1, **characterized in that** the fibrin web is firmly combined with a biodegradable support material.

3. A flexible wound covering as claimed in claims 1 and 2, **characterized in that** the fibrin web contains 10 to 80% by weight of glycerol per unit of fibrin.

4. A process for the production of a flexible wound covering based on fibrin, **characterized in that** it comprises subjecting a fibrinogen solution to a single-stage or multistage dialysis against a physiologically compatible inorganic salt in the dialysis bath in an amount of up to 0.5 percent by weight, then forming a flexible fibrin web by action of an aqueous thrombin solution which contains a water-soluble potassium salt on the fibrinogen solution and subsequently subjecting this to freeze-drying.

5. The process as claimed in claim 4, **characterized in that** the formation of the fibrin web is carried out in the presence of a biodegradable support material onto which the fibrin web deposits as a firmly adhering layer.

6. The process as claimed in claim 5, **characterized in that** the biodegradable support material consists of natural or chemically modified collagen, keratin, gelatin, carbohydrates or cellulose derivatives.

7. The process as claimed in claim 5, **characterized in that** the biodegradable support material consists of a polymer from the group consisting of the polyhydroxycarboxylic acids, the polyesters, the polycyanoacrylates, the polyamino acids, the polyalcohols or the silicones.

8. The process as claimed in claims 4 to 7, **characterized in that** the fibrinogen solution also additionally contains factor XIII.

9. The process as claimed in claims 4 to 8, **characterized in that** the fibrinogen solution is dialyzed in one stage with a solution containing a complexing agent and a physiologically compatible inorganic salt.

10. The process as claimed in claims 4 to 8, **characterized in that** the fibrinogen solution is dialyzed in one stage with a solution containing a complexing agent and subsequently a physiologically compatible inorganic salt is added to the fibrinogen solution.

11. The process as claimed in claims 4 to 8, **characterized in that** the dialysis is carried out in two stages, the fibrinogen solution first being dialyzed with a solution of a complexing agent and then with a solution of a physiologically compatible inorganic salt.

12. The process as claimed in claims 4 to 11, **characterized in that** the complexing agent employed for the dialysis of the fibrinogen solution is an aqueous solution of an alkali metal salt of ethylenediaminetetraacetic acid (EDTA), of oxalic acid or of citric acid.

13. The process as claimed in claims 9 to 12, **characterized in that** the concentration of the complexing agent in the dialysis bath is 1 to 20 mM, preferably 1 to 5 mM.

14. The process as claimed in claims 9 to 11, **characterized in that** the physiologically compatible inorganic salt employed is NaCl.

15. The process as claimed in claim 4, **characterized in that** the water-soluble calcium salt is added in an amount from 0.5 to 40 mM.

16. The process as claimed in claims 4 to 15, **characterized in that** the fibrinogen is employed in an amount from 10 to 25 g/l and the thrombin is employed in an amount of less than 1000 I.U./ml.

17. The process as claimed in claims 4 to 16, **characterized in that** active compounds promoting wound healing are added to the fibrin web.

18. The process as claimed in claims 4 to 16, **characterized in that** glycerol is added to the fibrinogen in an amount of 0.2 to 1.0 percent by volume during the preparation of the fibrin web.

19. A flexible wound covering based on fibrin, **characterized in that** it is obtainable as claimed in claims 4 to 18.

20. The use of a wound covering as claimed in claims 1 to 3 and 19 for manufacturing a medicament for the hemostasis of internal and external wounds.

21. The use of a wound covering as claimed in claims 1 to 3 and 19 for a manufacturing support material for active compounds promoting wound healing.

22. A support material for active compounds promoting wound healing preparable from a wound covering as claimed in claims 1 to 3 and 19.

## Revendications

1. Pansement flexible à base de fibrine, **caractérisé en ce que** le pansement contient un matériau non tissé de fibrine, susceptible d'être obtenu à partir d'un fibrinogène, et susceptible d'être préparé par un procédé dans lequel le fibrinogène est pratiquement ou totalement débarrassé de constituants dialysables, par une dialyse en une ou plusieurs étapes contre un sel inorganique physiologiquement compatible dans le bain de dialyse en une quantité allant jusqu'à 0,5% en poids.

2. Pansement flexible selon la revendication 1, **caractérisé en ce que** le matériau non tissé de fibrine est lié de manière fixe à un matériau de support biodégradable.

3. Pansement flexible selon les revendications 1 et 2, **caractérisé en ce que** le matériau non tissé de fibrine contient de 10 à 80% en poids de glycérine par rapport à la quantité de fibrine.

4. Procédé de préparation d'un pansement flexible à base de fibrine, **caractérisé en ce que** l'on soumet une solution de fibrinogène à une dialyse en une ou plusieurs étapes contre un sel inorganique physiologiquement compatible en une quantité allant jusqu'à 0,5% en poids dans le bain de dialyse après quoi, par l'action d'une solution aqueuse de thrombine, contenant un sel de calcium soluble dans l'eau, on forme à partir de la solution de fibrinogène un film de fibrine flexible et on soumet ensuite ce dernier à une lyophilisation.

5. Procédé selon la revendication 4, **caractérisé en ce que** la formation du matériau non tissé de fibrine a lieu en présence d'un matériau de support biodégradable, sur lequel le matériau non tissé de fibrine se dépose sous la forme d'une couche adhérente.

6. Procédé selon la revendication 5, **caractérisé en ce que** le matériau de support biodégradable est constitué de collagène naturel ou chimiquement modifié, de kératine, de gélatine, de carbohydrates ou de dérivés de cellulose.

7. Procédé selon la revendication 5, **caractérisé en ce que** le matériau de support biodégradable est constitué d'un polymère choisi parmi le groupe formé par les poly(acides hydroxycarboxyliques), les polyesters, les polycyanoacrylates, les polyacides aminés, les polyalcools ou les silicones.

8. Procédé selon les revendications 4 à 7, **caractérisé en ce que** la solution de fibrine contient en outre du facteur XIII.

9. Procédé selon les revendications 4 à 8, **caractérisé en ce que** la solution de fibrinogène est dialysée en une étape contre une solution contenant un agent complexant et un sel inorganique physiologiquement compatible.

10. Procédé selon les revendications 4 à 8, **caractérisé en ce que** la solution de fibrine est dialysée en une étape contre une solution contenant un agent complexant et que l'on ajoute ensuite à la solution de fibrinogène un sel inorganique physiologiquement compatible.

11. Procédé selon les revendications 4 à 8, **caractérisé en ce que** la dialyse est réalisée en deux étapes, la solution de fibrinogène étant dialysée d'abord contre une solution d'un agent complexant, et ensuite contre une solution d'un sel inorganique physiologiquement compatible.

12. Procédé selon les revendications 4 à 11, **caractérisé en ce que** l'on met en oeuvre en tant qu'agent complexant une solution aqueuse d'un sel de métal alcalin de l'acide éthylènediamine tétracétique (EDTA), de l'acide oxalique ou de l'acide citrique pour la dialyse de la solution de fibrinogène.

13. Procédé selon les revendications 9 à 12, **caractérisé en ce que** la concentration de l'agent complexant dans le bain de dialyse est de 1 à 20 mM, de préférence de 1 à 5 mM.

14. Procédé selon les revendications 9 à 11, **caractérisé en ce que** l'on met en oeuvre en tant que sel inorganique physiologiquement compatible du NaCl.

15. Procédé selon la revendication 4, **caractérisé en ce que** le sel de calcium soluble dans l'eau est ajouté en une quantité de 0,5 à 40 mM.

16. Procédé selon les revendications 4 à 15, **caractérisé en ce que** le fibrinogène est mis en oeuvre en une quantité de 10 à 25 g/l et la thrombine en une quantité de moins de 1000 U.I./ml.

17. Procédé selon les revendications 4 à 16, **caractérisé en ce que** le matériau non-tissé de fibrine est additionné d'agents actifs favorisant la guérison de blessures.

18. Procédé selon les revendications 4 à 16, **caractérisé en ce que**, lors de la préparation du matériau non-tissé de fibrine, on ajoute au fibrinogène de la glycérine en une quantité de 0,2 à 1% en volume.

19. Pansement flexible à base de fibrine, **caractérisé en ce qu'**il est susceptible d'être obtenu selon les revendications 4 à 18.

20. Utilisation d'un pansement selon les revendications 1 à 3 et 19 pour la préparation d'un médicament pour l'hémostase de blessures internes et externes.

21. Utilisation d'un pansement selon les revendications 1 à 3 et 19 pour la préparation d'un matériau de support pour les agents actifs favorisant la guérison de blessures.

22. Matériau de support pour les agents actifs favorisant la guérison de blessures, susceptible d'être obtenu à partir d'un pansement selon les revendications 1 à 3 et 19.
